# EUROPÄISCHE PATENTANMELDUNG

(11) **EP 1 634 862 A1**
(43) Veröffentlichungstag der Anmeldung: **15.03.2006**
(21) Anmeldenummer: 05018967.9
(22) Anmeldetag: 01.09.2005
(51) Int. Cl.: C07C 1/24

(54) **Verfahren zur Dehydratisierung von 2-Methylpentan-2,4-diol**

(30) Priorität: 11.09.2004 DE 102004043940
(71) Anmelder: Celanese Chemicals Europe GmbH, 61476 Kronberg (DE)
(72) Erfinder: Springer, Helmut, 46539 Dinslaken (DE)

(57) **Zusammenfassung**

Die vorliegende Erfindung betrifft ein Verfahren zur Dehydratisierung von 2-Methylpentandiol-2,4 zu einem Gemisch aus 2-Methyl-1,3-pentadien und 4-Methyl-1,3-pentadien bei erhöhter Temperatur in Gegenwart eines Säurekatalysators unter Verwendung eines Polyglykolethers als Wärmeträger, wobei ein Polyglykolether, enthaltend 80 bis 100 Gew.-% eines Polyethylenglykoldimethylethers der allgemeinen Formel [CH₃(OCH₂CH₂)ₙOCH₃ mit n = 2-8] und 0 bis 20 Gew.-% eines Polyethylenglykolmonomethylethers der allgemeinen Formel [CH₃(OCH₂CH₂)ₙOH mit n = 2-8], jeweils bezogen auf die Gesamtmasse an Polygkykolether, verwendet wird.

## Beschreibung

Die vorliegende Erfindung betrifft ein Verfahren zur Dehydratisierung von 2-Methylpentan-2,4-diol zu einem Gemisch aus 2-Methyl-1,3-pentadien und 4-Methyl-1,3-pentadien unter saurer Katalyse in einem Polyglykolether als Wärmeträger.

Es ist bekannt, durch Dehydratisierung von 2-Methylpentan-2,4-diol ein Gemisch aus 2-Methyl-1,3-Pentadien und 4-Methyl-1,3-Pentadien, nachfolgend Methylpentadien genannt, herzustellen. Methylpentadien besitzt als Ausgangsprodukt für die Herstellung von Riechstoffen eine besondere Bedeutung. Beispielsweise gelangt man durch eine Diels-Alder-Reaktion mit geeigneten olefinisch ungesättigten Verbindungen zu substituierten Cyclohexen-Derivaten, die wiederum für die Herstellung von Riechstoffkompositionen eine hohe Bedeutung erlangt haben.

Das Ausgangsprodukt 2-Methylpentan-2,4-diol ist nach bekannten Verfahren durch Kondensation von Aceton zum Diacetonalkohol mit nachfolgender Hydrierung gemäß GB 572,602 zugänglich. Nach GB 572,602 erfolgt die Dehydratisierung in Gegenwart von lod oder Chlorwasserstoff. Allerdings bereitet der Einsatz dieser flüchtigen Dehydratisierungskatalysatoren Probleme, beispielsweise sind Korrosionsprobleme an den Anlagenteilen zu erwarten.

Ein weiteres Verfahren zur Dehydratisierung von 2-Methylpentan-2,4-diol ist aus der JP-A-61-57525 bekannt. Bei diesem Verfahren verwendet man als Dehydratisierungskatalysator eine saure Verbindung, die in einem bestimmten Glykol oder in einem bestimmten Glykolether gelöst vorgelegt wird. In diese Mischung wird 2-Methylpentan-2,4-diol bei erhöhter Temperatur in einem Bereich von 90 bis 200°C kontinuierlich eingetragen. Offenbart wird auch die diskontinuierliche Verfahrensführung, bei der das katalysatorhaltige Lösungsmittel zusammen mit 2-Methylpentan-2,4-diol zunächst vorgelegt und dann erhitzt wird. Als Glykole werden beispielsweise Diethylenglykol oder Propylenglykol und als Glykolether Diethylenglykolmonobutylether, Diethylenglykoldibutylether oder Tetraethylenglykolmonomethylether verwendet. Das gewünschte Dehydratisierungsprodukt und Reaktionswasser wird aus dem Reaktionssystem abdestilliert und nach Wasserabtrennung gereinigt. Als saure Katalysatoren eignen sich Mineralsäuren oder organische Säuren wie Schwefelsäure, Phosphorsäure oder p-Toluolsulfonsäure sowie deren sauren Salze. Das bekannte Verfahren ist durch den Einsatz eines bestimmten Glykols oder Glykolethers charakterisiert. Die vorteilhafte Wirkung der Glykole als Lösungsmittel wird mit ihrem Lösevermögen gegenüber hochsiedenden Kondensationsprodukten gedeutet, die die Aktivität des sauren Katalysators beeinträchtigen können.

Die SU-A1-1,759,825 behandelt ebenfalls ein Verfahren zur Dehydratisierung von 2-Methylpentan-2,4-diol unter Verwendung bestimmter Glykole, nämlich Ethylenglykol, Diethylenglykol oder Triethylenglykol als hochsiedendes Lösungsmittel. Die katalytisch aktive Verbindung wird durch Auflösen von Kaliumhydroxid in dem Glykol bei einer Temperatur von 20-85°C und anschlie-ßender Umsetzung mit Schwefelsäure hergestellt. Die eigentliche Dehydratisierungsreaktion wird nach Zugabe von 2-Methylpentan-2,4-diol diskontinuierlich durchgeführt und erfolgt in Gegenwart von Hydrochinon, wobei das gewünschte Dehydratisierungsprodukt aus dem Reaktionsgemisch abdestilliert wird. Gegenüber dem Verfahren aus JP-A1-61-57525 lässt sich die Methylpentadien-Ausbeute und der Anteil an dem gewünschten 2-Methyl-1,3-pentadien verbessern, wenn ein Molverhältnis von 2-Methylpentan-2,4-diol:Kaliumhydroxid:Schwefelsäure:Glykol = 1:(0,10-0,25):(0,10-0,25):(0,2-1,0) eingehalten wird und wenn man als Glykol Ethylenglykol, Diethylenglykol oder Triethylenglykol einsetzt. SU-A1-1,759,825 berichtet bei der diskontinuierlich durchgeführten Dehydratisierung über eine Ausbeuteerhöhung an Methylpentadien auf 63-72% mit einem Gehalt an 2-Methyl-1,3-pentadien von 75-80%. SU-A1-1,759,825 weist ebenfalls darauf hin, dass die Verwendung von Ethylenglykolmonophenylether keinen vorteilhaften Effekt zeigt.

Der Stand der Technik erfordert ein aufwendiges Herstellverfahren für den sauren Katalysator sowie den Einsatz von bestimmten Glykolen in reiner Form als Lösungsmittel. Ebenfalls ist ein exaktes Molververhältnis zwischen den Reaktionskomponenten einzuhalten, um eine ausreichende Dehydratisierungsreaktion zu gewährleisten.

Es wurde nun überraschenderweise gefunden, dass sich 2-Methylpentan-2,4-diol in hohen Ausbeuten zu einem Gemisch aus 2-Methyl-1,3-pentadien und 4-Methyl-1,3-pentadien (Methylpentadien) dehydratisieren lässt, wenn man zunächst wässrige Lösungen saurer Salze von Mineralsäuren in technisch verfügbare Polyglykolether als Wärmeträger einträgt, und anschließend 2-Methylpentan-2,4-diol mit der so hergestellten Lösung bei erhöhter Temperatur behandelt.

Die Erfindung besteht daher in einem Verfahren zur Dehydratisierung von 2-Methylpentan-diol-2,4 zu einem Gemisch aus 2-Methyl-1,3-pentadien und 4-Methyl-1,3-pentadien in Gegenwart eines Säurekatalysators unter Verwendung eines Polyglykolethers als Wärmeträger, dadurch gekennzeichnet, dass man eine wässrige Lösung eines sauren Salzes einer Mineralsäure in einen Polyglykolether, enthaltend 80 bis 100 Gew.-% eines Polyethylenglykoldimethylethers der allgemeinen Formel [CH₃(OCH₂CH₂)ₙOCH₃ mit n = 2-8] und 0 bis 20 Gew.-% eines Polyethylenglykolmonomethylethers der allgemeinen Formel [CH₃(OCH₂CH₂)ₙOH mit n = 2-8], jeweils bezogen auf die Gesamtmasse an Polygkykolether, und wobei der Rest auf 100 Gew.-% im wesentlichen aus Hochsiedern besteht, einträgt und 2-Methylpentan-2,4-diol mit der so hergestellten Lösung bei erhöhter Temperatur behandelt.

Bei dem erfindungsgemäßen Verfahren können in einfacher Weise wässrige Lösungen kommerziell verfügbarer saurer Salze von Mineralsäuren eingesetzt werden. Beispiele solcher saurer Salze sind Alkali- oder Erdalkalisalze, wie Kalium- oder Natriumhydrogensulfat, Kalium- oder Natriumdihydrogenphosphat sowie Dikalium- oder Dinatriumhydrogenphosphat. Vorzugsweise verwendet man das kostengünstig verfügbare Kaliumhydrogensulfat. Üblicherweise setzt man eine wässrige Lösung mit einem Gehalt von 5 bis 25 Gew.-% an saurem Salz ein.

Besondere Bedeutung bei dem erfindungsgemäßen Verfahren kommt dem hochsiedenden Lösungsmittel, das man auch als Wärmeträger bezeichnet, zu. Überraschenderweise haben sich technisch verfügbare Polyglykolether als besonders brauchbar erwiesen, in denen verschiedene Polyethylenglykoldimethylether im Gemisch vorliegen, gegebenenfalls mit einem Anteil an Polyethylenglykolmonomethylethern. Dabei sind solche Gemische, enthaltend Polyethylenglykoldimethylether der allgemeinen Formel [CH₃(OCH₂CH₂)ₙOCH₃ mit n = 2-8] mit 80 bis 100 Gew.-% und Polyethylenglykolmonomethylether der allgemeinen Formel [CH₃(OCH₂CH₂)ₙOH mit n = 2-8] mit 0 bis 20 Gew.-%, jeweils bezogen auf die Gesamtmasse an Polyglykolether, zu verwenden. Der Rest auf 100 Gew.-% besteht im wesentlichen aus Hochsiedern. Besonders geeignet sind Gemische mit Polyethylenglykoldimethylethern, bei denen der Dimethylethergehalt, normiert auf 100 %, sich wie folgt aufteilt, nicht mehr als 65 Gew.-% Triethylenglykoldimethylether (n = 3), nicht mehr als 75 Gew.-% Tetraethylenglykoldimethylether ( n = 4) und nicht mehr als 20 Gew.-% Pentaethylenglykoldimethylether (n = 5) aufweist. Der Rest auf 100 Gew.-% besteht im wesentlichen aus Hexa-, Hepta- und Octaethylenglykoldimethylethern sowie Hochsiedern. Derartige Produkte sind kommerziell verfügbar, beispielsweise die Produkte PEG-DME 180 und PEG-DME 200 der Firma Clariant GmbH.

Überraschenderweise führt die Verwendung von reinen Polyethylenglykoldimethylethern, beispielsweise von reinem Triethylenglykoldimethylether als hochsiedendes Lösungsmittel oder Wärmeträger zu geringeren Ausbeuten an Methylpentadien.

Man führt die Dehydratisierung von 2-Methyl-2,4-pentandiol zweckmäßigerweise nach einem kontinuierlichen Verfahren durch, wobei man 2-Methyl-2,4-pentandiol in den aufgeheizten, Katalysator enthaltenden Wärmeträger über die Zeit zugibt und gleichzeitig die Spaltprodukte und Reaktionswasser abdestilliert. Die diskontinuierliche Arbeitsweise, nach der zunächst die gesamte Menge an 2-Methyl-2,4-pentandiol dem Wärmeträger mit dem darin enthaltenen Katalysator zugesetzt wird und nach der anschließend der Reaktionsansatz aufgeheizt wird und die Spaltprodukte abdestilliert werden, ist nicht ausgeschlossen, sie ist jedoch die weniger bevorzugte Variante.

Bei der bevorzugten kontinuierlichen Arbeitsweise wird zunächst der Wärmeträger vorgelegt und mit der wässrigen Lösung des sauren Salzes der Mineralsäure vermischt. Der Ansatz wird auf eine Temperatur von 110 bis 200°C aufgeheizt, wobei zugesetztes und im Wärmeträger vorhandenes Wasser abdestilliert. Das saure Salz fällt in fein kristalliner Form aus und lässt sich auf einfache Weise in dem Wärmeträger suspendieren, beispielsweise durch Rühren. Anschließend wird die Temperatur in dem Wärmeträger auf 120 bis 200°C, vorzugsweise auf 160 bis 190°C, eingestellt und 2-Methyl-2,4-pentandiol wird mit einer zeitlichen Rate eingetragen. Zeitgleich mit dem Eintrag von 2-Methyl-2,4-pentandiol in den Wärmeträger werden die Spaltprodukte zusammen mit dem gebildeten Wasser aus dem Wärmeträger abdestilliert. Die Dehydratisierung von 2-Methyl-2,4-pentandiol erfolgt im allgemeinen unter Normaldruck, die Anwendung von verminderten Drücken ist jedoch nicht ausgeschlossen, um beispielsweise Methylpentadien schneller aus der heißen Reaktionszone abzuführen.

Die abdestillierten Spaltprodukte werden in einer Vorlage gesammelt und von dem sich abscheidenden Wasser getrennt. Anschließend wird aus dem organischen Rohprodukt Methylpentadien destillativ abgetrennt.

Das Mengenverhältnis zwischen dem vorgelegten Wärmeträger und dem zugegebenen 2-Methyl-2,4-pentandiol, sowie die Zuführrate an 2-Methyl-2,4-pentandiol, falls man nach dem kontinuierlichen Verfahren arbeitet, kann über einen weiten Bereich variiert werden und ist für die Durchführung des erfindungsgemäßen Verfahrens nicht kritisch. Sie richtet sich nach den apparativen Gegebenheiten und kann durch Routinearbeiten optimiert werden.

Auch die Menge an in dem Wärmeträger suspendierten Katalysator kann über einen weiten Bereich variiert werden und durch Routineversuche ermittelt werden. Zu geringe Mengen an Katalysator bewirken keine ausreichende Spaltung mehr und zu hohe Mengen an Katalysator zeigen keinen positiven Effekt mehr sondern verteuern unnötig das Verfahren. Im allgemeinen setzt man pro 100 Gewichtsteilen Wärmeträger 5 bis 20, vorzugsweise 10 bis 15 Gewichtsteile Säurekatalysator, berechnet als Reinsubstanz, in Form einer wässrigen Lösung zu.

Das erfindungsgemäße Verfahren ergibt auf einfache Weise organische Rohprodukte mit einem Gehalt an Methylpentadien (2-Methyl-1,3-pentadien und 4-Methyl-1,3-pentadien) von 65-70 Gew.-%. Bezogen auf die Einsatzmenge an 2-Methyl-2,4-pentandiol liegt die destillativ nachgewiesene Ausbeute an Methylpentadien zwischen 58-63%.

Im Gegensatz zu dem aus dem Stand der Technik SU-A1-1 759 825 bekannten Verfahren zeichnet sich das beanspruchte Verfahren durch eine besonders einfache Arbeitsweise aus. Der Katalysator braucht nicht erst in einer separaten Stufe hergestellt werden, sondern er kann ausgehend von kommerziell verfügbaren Verbindungen direkt als wässrige Lösung eingesetzt werden. Auch die aufwendige Einstellung des komplizierten Molverhältnisses von 2-Methylpentan-2,4-diol : Kaliumhydroxid : Schwefelsäure : Glykol von 1 : (0,10 - 0,25) : (0,10 - 0,25) : (0,2 - 1,0) ist nach dem erfindungsgemäßen Dehydratisierungsverfahren nicht erforderlich.

Überraschenderweise zeigt sich, dass die Ausbeute an Methylpentadien von dem eingesetzten Wärmeträger abhängt. Während bei dem Einsatz von reinen Polyglykolethern Minderausbeuten beobachtet werden, lässt sich die Methylpentadien-Ausbeute erhöhen, wenn Polyglykolether in technischer Qualität, die in Form von Gemischen vorliegen, verwendet werden.

Im folgenden wird das erfindungsgemäße Verfahren anhand einiger Beispiele näher erläutert, es ist jedoch nicht auf die beschriebenen Ausführungsformen beschränkt.

### Beispiele

### 1. Dehydratisierung von 2-Methyl-2,4-pentandiol

Die Apparatur zur Flüssigphasen-Dehydratisierung von 2-Methyl-2,4-pentandiol bestand aus einem 500 ml-Rundkolben, ausgestattet mit einem Rührer, Innenthermometer sowie einer 30 cm-Vigreuxkolonne mit Claisenbrücke und Vorlage.

Im 500 ml-Rundkolben wurden als Wärmeträger 300,0 g Triethylenglykoldimethylether technischer Qualität (PEG-DME 180 der Firma Clariant GmbH) mit 53 Gew.-% Triethylenglykoldimethylether, 26 Gew.-% Tetraethylenglykoldimethylether, 8 Gew.-% Pentaethylenglykoldimethylether und 13 Gew.-% Rest (überwiegend Monomethylether) vorgelegt und mit einer wässrigen Lösung von 40,8 g Kaliumhydrogensulfat in 80,0 g Wasser versetzt. Unter Rühren wurde das Gemisch auf 180°C erwärmt, wobei das eingesetzte Wasser nahezu quantitativ abdestillierte. Dabei fiel das Kaliumhydrogensulfat fein kristallin aus und wurde unter Rühren in dem Wärmeträger suspendiert. In die Suspension wurden stündlich 80,0 g 2-Methyl-2,4-pentandiol gepumpt. Bei einer Kopftemperatur von 89°C destillierten die sich bildenden Spaltprodukte ab und wurden in einer Vorlage gesammelt. Nach Abtrennung des Reaktionswassers erhielt man ein organisches Rohprodukt folgender Zusammensetzung:

| Gaschromatographische Analyse (in Flächen-%): | |
|---|---|
| Vorlauf | 0,7 |
| Isobuten | 7,5 |
| 2-Methyl-1,3-pentadien* | 69,6 |
| 2-Methylpent-2-en-4-ol | 9,1 |
| Sonstige | 13,1 |

| | |
|---|---|
| *einschließlich 4-Methyl-1,3-pentadien | |

Die destillative Aufarbeitung des Rohprodukts an einer 24 Bödenkolonne bei Normaldruck ergab folgendes Ergebnis

**Tabelle 1: Destillative Aufarbeitung von rohem Methylpentadien**

| Fraktion | Temperatur (°C) | | Rücklaufverhältnis | Auswaage (Gew.-%) | |
|---|---|---|---|---|---|
| | Kopf | Sumpf | | org. Phase | Wasserphase |
| Kühlfalle | | | | 9,2 | |
| 1 | 65 | 84 | 5:1 1 | 3,9 | 0,2 |
| 2 | 67 | 85 | 5 : 1 | 1,5 | 0,1 |
| 3 | 80 | 135 | 2 : 1 | 56,0 | 1,1 |
| 4 | 122 | 180 | 1:1 1 | 21,8 | 0,5 |
| Rückstand | | | | 5,7 | |

**Tabelle 2: Gaschromatographische Analyse (in Flächen-%) der destillativ erhaltenen Fraktionen**

| Fraktion | Kühlfalle | 1 | 2 | 3 | 4 | Rückstand |
|---|---|---|---|---|---|---|
| Vorlauf | | 0,89 | 0,30 | 0,01 | 0,04 | |
| Isobuten | 100,0 | 11,71 | 4,87 | 0,14 | 0,66 | |
| 2-Methyl-1,3-pentadien* | | 76,81 | 85,26 | 97,82 | 6,15 | nicht bestimmt |
| 2-Methylpent-2-en-4-ol | | | | 0,01 | 40,96 | |
| Sonstige | | 10,59 | 9,57 | 2,02 | 52,19 | |

| | | | | | | |
|---|---|---|---|---|---|---|
| * einschließlich 4-Methyl-1,3-pentadien | | | | | | |

Schwefelgehalt: < 0,2 ppm 0,3 ppm <0,2 ppm 12 ppm

### 2. Vergleichsbeispiel

Es wurde analog Beispiel 1 gearbeitet mit der einzigen Ausnahme, dass als Wärmeträger anstelle von Triethylenglykoldimethylether technischer Qualität reiner Triethylenglykoldimethylether (Gehalt 99 Gew.-%) eingesetzt wurde. Das erhaltene organische Spaltprodukt wies nach gaschromatographischer Analyse (Angaben in Flächen-%) einen Gehalt von 2-Methyl-1,3-pentadien/4-Methyl-1,3-pentadien von 56 % aus und der Gehalt an 2-Methylpent-2-en-4-ol lag bei 26 %.

Wie das erfindungsgemäße Beispiel und das Vergleichsbeispiel belegen, hat die Natur des Wärmeträgers einen bedeutenden Einfluß auf den Dehydratisierungsgrad von 2-Methyl-2,4-pentandiol.

## Patentansprüche

1. Verfahren zur Dehydratisierung von 2-Methylpentan-diol-2,4 zu einem Gemisch aus 2-Methyl-1,3-pentadien und 4-Methyl-1,3-pentadien in Gegenwart eines Säurekatalysators unter Verwendung eines Polyglykolethers als Wärmeträger, **dadurch gekennzeichnet, dass** man eine wässrige Lösung eines sauren Salzes einer Mineralsäure in einen Polyglykolether, enthaltend 80 bis 100 Gew.-% eines Polyethylenglykoldimethylethers der allgemeinen Formel [CH₃(OCH₂CH₂)ₙOCH₃ mit n = 2-8] und 0 bis 20 Gew.-% eines Polyethylenglykolmonomethylethers der allgemeinen Formel [CH₃(OCH₂CH₂)ₙOH mit n = 2-8], jeweils bezogen auf die Gesamtmasse an Polyglykolether, und wobei der Rest auf 100 Gew.-% im wesentlichen aus Hochsiedern besteht, einträgt und 2-Methylpentan-2,4-diol mit der so hergestellten Lösung bei erhöhter Temperatur behandelt.

2. Verfahren gemäß Anspruch 1, **dadurch gekennzeichnet, dass** der Polyethylenglykoldimethylether, normiert auf 100 %, nicht mehr als 65 Gew.-% Triethylenglykoldimethylether, nicht mehr als 75 Gew.-% Tetraethylenglykoldimethylether und nicht mehr als 20 Gew.-% Pentaethylenglykoldimethylether enthält und wobei der Rest auf 100 Gew.-% im wesentlichen aus Hexa-, Hepta- und Octaethylenglykoldimethylethern sowie Hochsiedern besteht.

3. Verfahren gemäß Anspruch 1 oder 2, **dadurch gekennzeichnet, dass** man eine wässrige Lösung eines sauren Salzes einer Mineralsäure in den Polyglykolether einträgt, den Polyglykolether zunächst auf 110 bis 200°C aufheizt und vorhandenes Wasser abdestilliert, anschließend die Temperatur auf 120° bis 200°C einstellt, 2-Methylpentan-diol-2,4 zusetzt und gleichzeitig die Dehydratisierungsprodukte aus dem Polyglykolether abdestilliert.

4. Verfahren nach Anspruch 3, **dadurch gekennzeichnet, dass** nach Abdestillation des Wassers die Temperatur auf 160 bis 190°C eingestellt wird.

5. Verfahren nach einem oder mehreren der Ansprüche 1 bis 4, **dadurch gekennzeichnet, dass** als saure Salze einer Mineralsäure Alkali- oder Erdalkalisalze verwendet werden.

6. Verfahren nach Anspruch 5, **dadurch gekennzeichnet, dass** Kaliumhydrogensulfat, Natriumhydrogensulfat, Kaliumdihydrogenphosphat, Dikaliumhydrogenphosphat, Natriumdihydrogenphosphat oder Dinatriumhydrogenphosphat als saure Salze verwendet werden.

7. Verfahren nach einem oder mehreren der Ansprüche 1 bis 6, **dadurch gekennzeichnet, dass** die wässrige Lösung des sauren Salzes einer Mineralsäure einen Gehalt von 5 bis 25 Gew.-% an saurem Salz aufweist.

8. Verfahren nach einem oder mehreren der Ansprüche 1 bis 7, **dadurch gekennzeichnet, dass** man pro 100 Gewichtsteilen Wärmeträger 5 bis 20, vorzugsweise 10 bis 15 Gewichtsteile an Säurekatalysator, berechnet als Reinsubstanz, in Form einer wässrigen Lösung zugibt.
